(19) Europäisches Patentamt
European Patent Office
Office européen des brevets

(11) Veröffentlichungsnummer: **0 000 002**
**B1**

(12) # EUROPÄISCHE PATENTSCHRIFT

(45) Veröffentlichungstag der Patentschrift: **26.08.81**

(21) Anmeldenummer: **78100007.0**

(22) Anmeldetag: **01.06.78**

(51) Int. Cl.³: **C 07 D 307/12,**
C 07 D 407/12,
C 07 D 307/42 //A01N43/08

(54) **Tetrahydrofuran-Derivate, Verfahren zu ihrer Herstellung sowie ihre Verwendung als Herbizide.**

(30) Priorität: **01.06.77 DE 2724675**

(43) Veröffentlichungstag der Anmeldung:
**20.12.78 Patentblatt 78/1**

(45) Bekanntmachung des Hinweises auf die Patenterteilung:
**26.08.81 Patentblatt 81/34**

(84) Benannte Vertragsstaaten:
**BE CH DE FR GB NL SE**

(56) Entgegenhaltungen:
**FR - A - 1 370 678**
**NL - A - 7 712 277**

**JOURNAL OF THE AMERICAN CHEMICAL SOCIETY, Band *98* (1976) Seiten 1526—1537**

(73) Patentinhaber: **BAYER AG**
**Zentralbereich Patente, Marken und Lizenzen**
**D-5090 Leverkusen 1, Bayerwerk (DE)**

(72) Erfinder: **Schmidt, Thomas, Dr.**
**Wormser strasse 23**
**D-5600 Wuppertal 1 (DE)**
Erfinder: **Draber, Wilfried, Dr.**
**In den Birken 81**
**D-5600 Wuppertal 1 (DE)**
Erfinder: **Eue, Ludwig, Dr.**
**Paul-Klee-Strasse 36**
**D-090 Leverkusen 1 (DE)**
Erfinder: **Schmidt, Robert Rudolf, Dr.**
**Hahnenweg 5**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EPO 0 000 002 B1

Tetrahydrofuran-Derivate, Verfahren zu ihrer Herstellung sowie ihre Verwendung als Herbizide

Die Erfindung betrifft Tetrahydrofuran-Derivate, mehrere Verfahren zu ihrer Herstellung sowie ihre Verwendung als Herbizide, insbesondere als selektive Herbizide.

Es ist bereits bekannt geworden, daß Chloracetanilide, wie beispielsweise 2-Aethyl-6-methyl-N-(1'-methyl-2'-methoxyäthyl)chloracetanilid, als Herbizide, insbesondere zur Bekämpfung von grasartigen Unkräutern, verwendet werden können (vgl. DE—A—2 328 340). Diese Verbindungen sind in ihrer Selektivität jedoch nicht immer befriedigend.

Es wurden Tetrahydrofuran-Derivate der Formel I

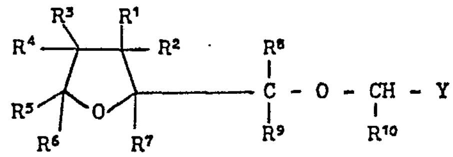

(I)

in welcher

$R^1$ bis $R^6$ gleich oder verschieden sind und für Wasserstoff, geradkettiges oder verzweigtes $C_{1-6}$-Alkyl, $C_{1-2}$-Halogenalkyl mit bis zu drei gleichen oder verschiedenen Halogenatomen (wobei als Halogene insbesondere Fluor, Chlor und Brom stehen), Alkoxyalkyl mit 1 oder 2 C-Atomen in jedem Alkylteil, sowie für jeweils gegebenenfalls durch Halogen, $C_{1-2}$-Alkyl, $C_{1-2}$-Alkoxy oder $C_{1-2}$-Halogenalkyl mit bis zu drei gleichen oder verschiedenen Halogenatomen (wie insbesondere Fluor und Chlor) substituiertes Phenyl oder Benzyloxyalkyl mit 1 oder 2 C-Atomen im Alkylteil stehen,

$R^5$ und $R^6$ auch gemeinsam für einen gesättigten carbocyclischen Ring mit insgesamt 3 bis 6 C-Atomen stehen,

$R^7$ für Wasserstoff, geradkettiges oder verzweigtes $C_{1-6}$-Alkyl, $C_{3-6}$-Cycloalkyl, $C_{2-4}$-Alkenyl, $C_{2-4}$-Alkinyl, Alkoxyalkyl mit 1 bis 2 C-Atomen in jedem Alkylteil, sowie für jeweils durch Halogen, $C_{1-2}$-Alkyl, $C_{1-2}$-Alkoxy, oder $C_{1-2}$-Halogenalkyl mit bis zu drei gleichen oder verschiedenen Halogenatomen (wie insbesondere Fluor und Chlor) substituiertes Phenyl oder Benzyloxyalkyl mit 1 oder 2 C-Atomen im Alkylteil steht,

$R^8$ und $R^9$ gleich oder verschieden sind und für Wasserstoff, geradkettiges oder verzweigtes $C_{1-6}$-Alkyl, $C_{2-4}$-Alkenyl, $C_{2-4}$-Alkinyl, $C_{3-6}$-Cycloalkyl, $C_{1-4}$-Alkoxy, sowie für jeweils gegebenenfalls durch Halogen, $C_{1-2}$-Alkyl, $C_{1-2}$-Alkoxy oder $C_{1-2}$-Halogenalkyl mit bis zu drei gleichen oder verschiedenen Halogenatomen (wie insbesondere Fluor und Chlor) substituiertes Phenyl, Benzyl oder Benzyloxy stehen,

$R^{10}$ für Wasserstoff, geradkettiges oder verzweigtes $C_{1-6}$-Alkyl, $C_{2-4}$-Alkenyl, $C_{2-4}$-Alkinyl, $C_{1-2}$-Halogenalkyl mit bis zu drei gleichen oder verschiedenen Halogenatomen (wobei als Halogene insbesondere Fluor, Chlor oder Brom stehen), sowie für gegebenenfalls durch Halogen, $C_{1-2}$-Alkyl, $C_{1-2}$-Alkoxy oder $C_{1-2}$-Halogenalkyl mit bis zu drei gleichen oder verschiedenen Halogenatomen (wie insbesondere Fluor und Chlor) substituiertes Phenyl steht und

Y für gegebenenfalls substituiertes Aryl mit 6 bis 10 C-Atomen (insbesondere für Phenyl und Naphthyl) steht, welches einen oder mehrere gleiche oder verschiedene der folgenden Substituenten tragen kann: die Halogene Fluor, Chlor oder Brom; Alkyl und Alkoxy mit jeweils 1 bis 4 C-Atomen; Halogenalkyl, Halogenalkoxy und Halogenalkylthio mit jeweils bis zu 4 C-Atomen und bis zu 5 Halogenatomen (insbesondere mit bis zu 2 C-Atomen und bis zu 3 gleichen oder verschiedenen Halogenatomen, wobei als Halogene insbesondere Fluor, Chlor und Brom stehen); gegebenenfalls durch Halogen, (insbesondere Fluor, Chlor oder Brom), Alkyl und Alkoxy mit jeweils 1 bis 2 C-Atomen sowie Halogenalkyl mit bis zu 2 C-Atomen und bis zu 3 gleichen oder verschiedenen Halogenatomen (wie insbesondere Fluor und Chlor), substituiertes Phenyl, Phenoxy oder Phenoxycarbonyl; Alkoxycarbonyl mit 1 bis 4 C-Atomen im Alkylteil; die Methylendioxy-Gruppe sowie den Tri-, Tetra- oder Pentamethylen-Rest; mit der Maßgabe, daß, falls Y für Phenyl steht, dieses substituiert sein muß, wenn $R^1$ bis $R^{10}$ Wasserstoff bedeuten,

aufgefunden.

Diese Tetrahydrofuran-Derivate weisen starke herbizide, insbesondere selektiv-herbizide Eigenschaften auf.

Weiterhin wurde gefunden, daß man die Tetrahydrofuran-Derivate der Formel I erhält, wenn man

(a) Alkoholate von 2-Hydroxymethyl-tetrahydrofuran-Derivaten der Formel II

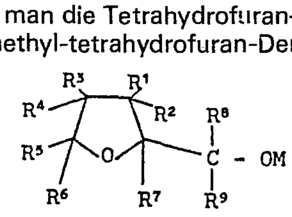

(II)

in welcher
$R^1$ bis $R^9$ die oben angegebene Bedeutung haben und
M für ein Alkali- oder Erdalkalimetall (insbesondere für Natrium und Kalium sowie für 1 Äquivalent Magnesium und Calcium) steht,
mit einer Verbindung der Formel III

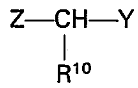

(III)

in welcher
$R^{10}$ und Y die oben angegebene Bedeutung haben und
Z für Halogen (insbesondere Chlor oder Brom), den Mesylat- oder Tosylat-Rest steht,
in an sich bekannter Weise, gegebenenfalls in Gegenwart eines Verdünnungsmittels umsetzt, oder wenn man

(b) Diole der Formel IV

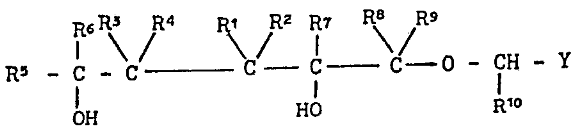

(IV)

in welcher
$R^1$ bis $R^{10}$ und
Y die oben angegebene Bedeutung haben,
in an sich bekannter Weise in Gegenwart eines sauren Katalysators und gegebenenfalls in Gegenwart eines Verdünnungsmittels erhitzt.

Verbindungen der Formel I, in denen entweder $R^1$ und $R^7$ oder $R^1$ und $R^4$ oder $R^4$ und $R^5$ für Wasserstoff stehen, können auch erhalten werden, wenn man

(c) Dihydrofuran-Derivate der Formeln Va, Vb und Vc

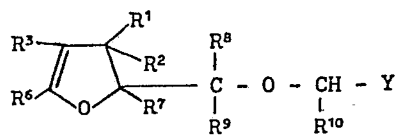

(Va)

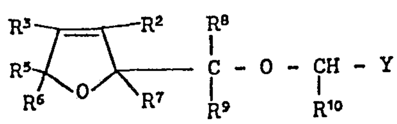

(Vb)

und/oder

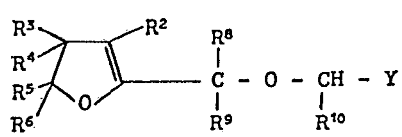

(Vc)

in welchen
$R^1$ bis $R^{10}$ und
Y die oben angegebene Bedeutung haben,
in an sich bekannter Weise in Gegenwart eines Katalysators und gegebenenfalls in Gegenwart eines Verdünnungsmittels mit Wasserstoff hydriert.

Verbindungen der Formel I, in denen $R^1$, $R^4$, $R^5$ und $R^7$ für Wasserstoff stehen, können auch erhalten werden, wenn man

(d) Furan-Derivate der Formel VI

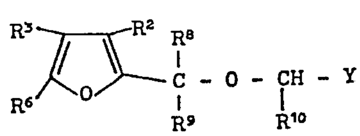

(VI)

in welcher
$R^2$, $R^3$, $R^6$, $R^8$, $R^9$, $R^{10}$ und Y die oben angegebene Bedeutung haben,
in an sich bekannter Weise in Gegenwart eines Katalysators und gegebenenfalls in Gegenwart eines Verdünnungsmittels mit Wasserstoff hydriert.

Ueberraschenderweise sind die erfindungsgemäßen Tetrahydrofuran-Derivate den bekannten Gräserbekämpfungsmitteln, wie beispielsweise 2 - Aethyl - 6 - methyl - N - (1' - methyl - 2' - methoxyäthyl) - chloracetanilid, in der herbiziden Wirkung überlegen und zeigen außerdem eine deutlich bessere Selektivität gegenüber wichtigen Kulturpflanzen. Die erfindungsgemäßen Wirkstoffe

stellen somit eine wesentliche Bereicherung der herbiziden Mittel, insbesondere der Gräserherbizide, dar.

Verwendet man das Natriumalkoholat des Tetrahydrofurfurylalkohols und 2-Fluorbenzylbromid als Ausgangsstoffe, so kann der Reaktionsablauf durch das folgende Formelschema wiedergegeben werden (Verfahrensvariante a):

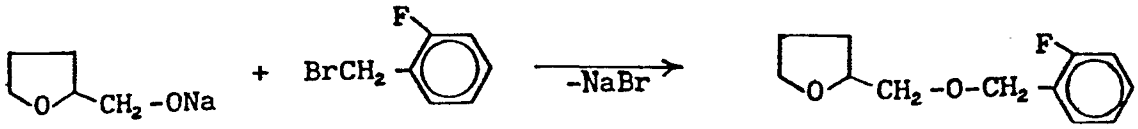


Verwendet man 1-(2-Fluorbenzyloxy)-pentan-2,5-diol als Ausgangsstoff und p-Toluolsulfonsäure als Katalysator, so kann der Reaktionsablauf durch das folgende Formelschema wiedergegeben werden (Verfahrensvariante b):

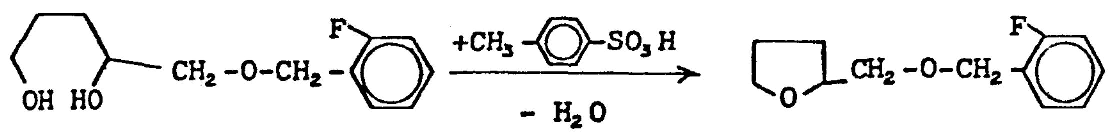


Verwendet man 2,3-Dihydro-2-(2-Fluorbenzyloxymethyl)-furan und Wasserstoff als Ausgangsstoffe, so kann der Reaktionsablauf durch das folgende Formelschema wiedergegeben werden (Verfahrens — variante c):

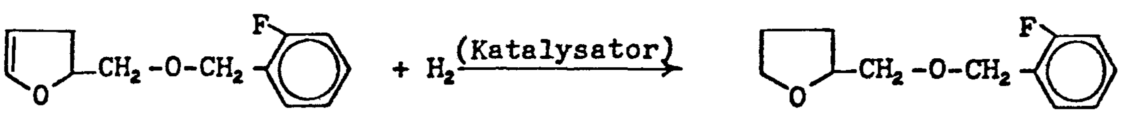


Verwendet man 2-[1-(2-Chlorbenzyloxy)-prop-1-yl]-furan und Wasserstoff als Ausgangsstoffe, so kann der Reaktionsablauf durch das folgende Formelschema wiedergegeben werden (Verfahrensvariante d):

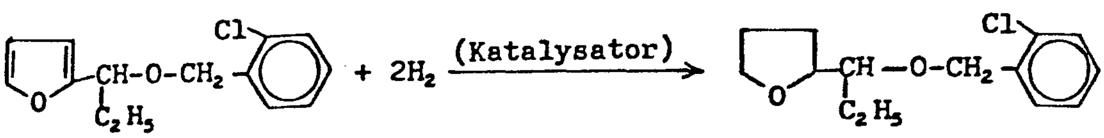


Die Alkoholate der Formel II sind bekannt oder lassen sich nach bekannten Methoden herstellen. Man erhält sie z.B., indem man die entsprechenden 2-Hydroxymethyl-tetrahydrofuran-Derivate mit geeigneten starken Basen, wie beispielsweise Alkali- bzw. Erdalkaliamiden, -hydriden oder -hydroxiden, in einem inerten Lösungsmittel umsetzt. Die genannten 2-Hydroxymethyl-tetrahydrofuran-Derivate sind ebenfalls bekannt oder lassen sich nach bekannten Methoden herstellen (vgl. u.a. H.Kröper, in Houben-Weyl, 'Methoden der organischen Chemie', Band 6/3, S.519ff [1965] sowie die dort zitierte Literatur).

Als Beispiele für die 2-Hydroxymethyl-tetrahydrofuran-Derivate, die den erfindungsgemäß als Ausgangsstoffe zu verwendenden Alkoholaten der Formel II zugrunde liegen, seien genannt:

2-Hydroxymethyl-tetrahydrofuran
2-(1-Hydroxyprop-1-yl)-tetrahydrofuran
2-(1-Hydroxyäthyl)-tetrahydrofuran
2-(3-Hydroxypent-3-yl)-tetrahydrofuran
2-(Hydroxy-phenyl-methyl)-tetrahydrofuran
2-(2-Hydroxyprop-2-yl)-tetrahydrofuran

Die weiterhin für die Verfahrensvariante (a) als Ausgangstoffe zu verwendenden Verbindungen sind durch die Formel III allgemein definiert.

Die Ausgangsstoffe der Formel III sind allgemein bekannte Verbindungen der organischen Chemie. Als Beispiele seien genannt:
Benzylchlorid, Benzylbromid, Benzylmesylat, Benzyltosylat, 2-Fluorbenzylbromid, 3-Fluorbenzylbromid, 4-Fluorbenzylbromid, 2-Chlorbenzylchlorid, 3-Chlorbenzylchlorid, 4-Chlorbenzylchlorid, 2-Brombenzylchlorid, 3-Brombenzylchlorid, 4-Brombenzylchlorid, 2-Methylbenzylchlorid, 3-Methylbenzylchlorid, 4-Methylbenzylchlorid, 2-Methoxybenzylchlorid, 3-Methoxybenzylchlorid, 4-Methoxybenzylchlorid, 2-Trifluormethylbenzylchlorid, 3-Trifluormethylbenzylchlorid, 4-Trifluormethylbenzylchlorid, 4-Phenylbenzylchlorid, 2,6-Difluorbenzylchlorid, 2,6-Dichlorbenzylchlorid, 2,4-Dichlorbenzylchlorid, 3,4-Dichlorbenzylchlorid, 2,5-Dichlorbenzylchlorid, 2,6-Dimethylbenzylchlorid, 2,4-Dimethylbenzylbromid, 3,4-Dimethylbenzylchlorid, 2,3-Dimethylbenzylchlorid, 3,4-Dioxymethylenbenzylchlorid, 2,6-Chlorfluorbenzylchlorid, 2-Fluor-5-chlorbenzylbromid, 2-Fluor-4-chlorbenzylbromid, 3-Chlor-4-fluorbenzylbromid, 3,4-Tetramethylenbenzylchlorid, 2-Methyl-6-chlorbenzylchlorid, 2-Methyl-6-fluorbenzylchlorid, 2-Fluor-3-methylbenzylchlorid, 2-Fluor-4-methylbenzylchlorid, 2-Fluor-5-methylbenzylchlorid, 2-Methyl-3-chlorbenzylchlorid, 2-Methyl-4-chlorbenzylchlorid, 2-Methyl-5-chlorbenzylchlorid, 2,4,5-Trichlorbenzylbromid, 2,4,6-Trichlorbenzylbromid, Diphenylmethylbromid, 1-Brom-1-phenyläthan, 1-Brom-1-(2-fluorphenyl)-äthan, 1-Brom-1-(2-methylphenyl)-äthan.

Die für die Verfahrensvariante (b) als Ausgangsstoffe zu verwendenden Diole sind durch die Formel IV allgemein definiert.

Die Diole der Formel IV sind bekannt bzw. lassen sie sich nach bekannten Methoden herstellen. Als Beispiele seien genannt:

1-Benzyloxy-pentan-2,5-diol
1-(2-Fluorbenzyloxy)-pentan-2,5-diol
1-(2-Chlorbenzyloxy)-pentan-2,5-diol
1-(2-Methylbenzyloxy)-pentan-2,5-diol
1-(2-Brombenzyloxy)-pentan-2,5-diol
1-(4-Fluorbenzyloxy)-pentan-2,5-diol
1-(2,6-Dichlorbenzyloxy)-pentan-2,5-diol
1-Benzyloxy-5,5-dimethyl-pentan-2,5-diol
1-(2-Fluorbenzyloxy)-5,5-dimethyl-pentan-2,5-diol
1-(2-Chlorbenzyloxy)-5,5-dimethyl-pentan-2,5-diol
1-(2-Methylbenzyloxy)-5,5-dimethyl-pentan-2,5-diol
1-(2-Brombenzyloxy)-5,5-dimethyl-pentan-2,5-diol
1-(4-Fluorbenzyloxy)-5,5-dimethyl-pentan-2,5-diol
1-(2,6-Dichlorbenzyloxy)-5,5-dimethyl-pentan-2,5-diol
1-Benzyloxy-2,5,5-trimethyl-pentan-2,5-diol
1-(2-Fluorbenzyloxy)-2,5,5-trimethyl-pentan-2,5-diol
1-(2-Chlorbenzyloxy)-2,5,5-trimethyl-pentan-2,5-diol
1-(2-Methylbenzyloxy)-2,5,5-trimethyl-pentan-2,5-diol
1-(2-Brombenzyloxy)-2,5,5-trimethyl-pentan-2,5-diol
1-(4-Fluorbenzyloxy)-2,5,5-trimethyl-pentan-2,5-diol
1-(2,6-Dichlorbenzyloxy)-2,5,5-trimethyl-pentan-2,5-diol
1-Benzyloxy-2-äthyl-5,5-dimethyl-pentan-2,5-diol
1-(2-Fluorbenzyloxy)-2-äthyl-5,5-dimethyl-pentan-2,5-diol
1-(2-Chlorbenzyloxy)-2-äthyl-5,5-dimethyl-pentan-2,5-diol
1-(2-Methylbenzyloxy)-2-äthyl-5,5-dimethyl-pentan-2,5-diol
1-(2-Brombenzyloxy)-2-äthyl-5,5-dimethyl-pentan-2,5-diol
1-(4-Fluorbenzyloxy)-2-äthyl-5,5-dimethyl-pentan-2,5-diol
1-(2,6-Dichlorbenzyloxy)-2-äthyl-5,5-dimethyl-pentan-2,5-diol

Die für die Verfahrensvariante (c) als Ausgangsstoffe zu verwendenden Dihydrofuran-Derivate sind durch die Formeln Va, Vb und Vc allgemein definiert.

Die Dihydrofuran-Derivate der Formeln Va, Vb und Vc sind noch nicht bekannt, Man erhält sie jedoch auf einfache Weise, wenn man entsprechend der Verfahrensvariante (a) Alkoholate von 2-Hydroxymethyl-dihydrofuran-Derivaten der Formeln VIIa, VIIb und VIIc

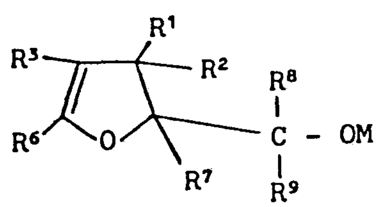

(VIIa)

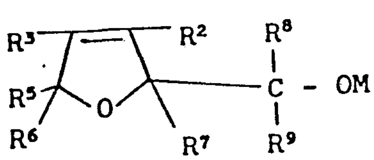

(VIIb)

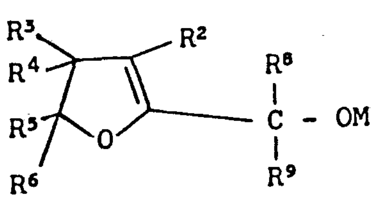

(VIIc)

in welchen
$R^1$ bis $R^9$ und
M die oben angegebene Bedeutung haben,
mit einer Verbindung der Formel III gegebenenfalls in Gegenwart eines Verdünnungsmittels umsetzt.

Die für die Verfahrensvariante (d) als Ausgangsstoffe zu verwendenden Furan-Derivate sind durch die Formel VI allgemein definiert.

Die Furan-Derivate der Formel VI sind noch nicht bekannt. Man erhält sie jedoch auf einfache Weise, wenn man entsprechend der Verfahrensvariante (a) Alkoholate von 2-Hydroxymethyl-furan-Derivaten der Formel VIII

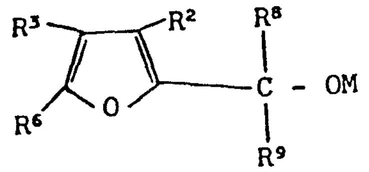

(VIII)

in welcher
$R^2$, $R^3$, $R^6$, $R^8$, $R^9$ und M die oben angegebene Bedeutung haben,
mit einer Verbindung der Formel III gegebenenfalls in Gegenwart eines Verdünnungsmittels umsetzt.

Als Ausgangsstoffe der Formel VI seien beispielsweise genannt:

2-(2-Fluorbenzyloxymethyl)-furan
2-[1-(2-Fluorbenzyloxy)-äth-1-yl]-furan
2-[1-(2-Chlorbenzyloxy)-prop-1-yl]-furan
2-[1-(2-Fluorbenzyloxy)-prop-1-yl]-furan
2-[1-(2-Fluorbenzyloxy)-äth-1-yl]-furan
2-[1-(2-Methylbenzyloxy)-äth-1-yl]-furan
2-[1-(Benzyloxy)-äth-1-yl]-furan
2-[3-(2-Fluorbenzyloxy-pent-3-yl]-furan
2-[3-(2-Methylbenzyloxy)-pent-3-yl]-furan
2-[3-(2-Chlorbenzyloxy)-pent-3-yl]-furan
2-[1-(2-Chlorbenzyloxy)-äth-1-yl]-furan
2-[$\alpha$-(2-Fluorbenzyloxy)-benzyl]-furan
2-[$\alpha$-(2-chlorbenzyloxy)-benzyl]-furan
2-[2-(2-Fluorbenzyloxy)-prop-2-yl]-furan
2-($\alpha$-Phenylbenzyloxy-methyl)-furan

Die Alkoholate der Formeln VIIa, VIIb, VIIc und VIII sind bekannt oder lassen sich nach bekannten Methoden herstellen. Man erhält sie z.B., indem man die entsprechenden 2-Hydroxymethyldihydrofuran-Derivate bzw. 2-Hydroxymethyl-furan-Derivate mit geeigneten starken Basen, wie beispielsweise Alkali- oder Erdalkaliamiden, -hydriden oder -hydroxiden, in einem inerten Lösungsmittel umsetzt. Die genannten 2-Hydroxymethyl-Derivate sind ebenfalls bekannt oder lassen sich nach bekannten Methoden herstellen (vgl. u.a. H.Kröper in Houben-Weyl, 'Methoden der organischen Chemie', Band 6/3, S.519ff [1965] sowie die dort zitierte Literatur).

Für die erfindungsgemäße Umsetzung gemäß Verfahrensvariante (a) kommen als Verdünnungsmittel vorzugsweise inerte organische Lösungsmittel infrage. Hierzu gehören vorzugsweise Aether, wie Diäthyläther, Tetrahydrofuran oder Dioxan, aromatische Kohlenwasserstoffe, wie Benzol oder Toluol, in einzelnen Fällen auch chlorierte Kohlenwasserstoffe, wie Chloroform, Methylenchlorid oder Tetrachlorkohlenstoff.

Die Reaktionstemperaturen können bei der Verfahrensvariante (a) in einem größeren Bereich variiert werden. Im allgemeinen arbeitet man zwischen 0 und 120°C, vorzugsweise bei 20 bis 100°C. Bei der Durchführung der erfindungsgemäßen Verfahrensvariante (a) arbeitet man vorzugsweise in molaren Mengen. Es ist aber auch möglich, die Alkoholate der Formel II oder die Verbindungen der Formel III im Ueberschuß bis zu 1 Mol einzusetzen. Zur Isolierung der Endprodukte wird das Reaktionsgemisch mit Wasser versetzt, die organische Phase abgetrennt und in üblicher Weise aufgearbeitet und gereinigt. In einzelnen Fällen kann das Endprodukt auch direkt nach dem Lösungsmittel aus dem Rekationsprodukt abdestilliert werden.

Nach einer bevorzugten Ausführungsform wird zweckmäßigerweise so verfahren, daß man von einem 2-Hydroxymethyl-tetrahydrofuran-Derivat ausgeht, letzteres in einem geeigneten inerten Lösungsmittel mittels Alkalimetall-hydrid oder -amid in das Alkalimetall-alkoholat der Formel II überführt, und letzteres ohne Isolierung sofort mit einer Verbindung der Formel III umsetzt und somit die erfindungsgemäßen Verbindungen der Formel I in einem Arbeitsgang erhält. Dabei kann die Verbindung der Formel III auch bereits vor der Herstellung des Alkoholats dem Reaktionsgemisch zugesetzt werden.

Nach einer weiteren bevorzugten Ausführungsform werden zweckmäßigerweise die Herstellung des Alkoholats der Formel II sowie die erfindungsgemäße Umsetzung nach Verfahren (a) in einem

Zweiphasensystem, wie z.B. wässrige Natron- oder Kalilauge/Toluol oder Methylenchlorid, unter Zusatz eines Phasen-Transfer-Katalysators, wie beispielsweise Ammonium- oder Phosphoniumverbindungen durchgeführt.

Die erfindungsgemäße Umsetzung gemäß Verfahrensvariante (b) wird vorzugsweise ohne Lösungsmittel durchgeführt.

Die erfindungsgemäße Umsetzung gemäß Verfahrensvariante (b) wird in Gegenwart eines sauren Katalysators durchgeführt. Man kann alle üblicherweise verwendbaren anorganischen und organischen sauren Katalysatoren einsetzen. Hierzu gehören vorzugsweise organische Säuren, wie p-Toluolsulfonsäure, anorganische Säuren, wie Salzsäure und Schwefelsäure, sowie Metallhalogenide, wie Aluminiumchlorid.

Die Reaktionstemperaturen können bei der Verfahrensvariante (b) in einem größeren Bereich variiert werden. Im allgemeinen arbeitet man zwischen 80 und 250°C, vorzugsweise zwischen etwa 100 und 220°C.

Zur Isolierung der Endprodukte wird bei der Verfahrensvariante (b) das Reaktionsgemisch im Vakuum destilliert und anschließend das Wasser in üblicher Weise abgetrennt.

Für die erfindungsgemaßen Umsetzungen gemäß Verfahrensvarianten (c) und (d) kommen bei Verwendung eines Verdünnungsmittels vorzugsweise inerte organische Lösungsmittel infrage. Hierzu gehören vorzugsweise Alkohole, wie Methanol und Aethanol sowie Aether, wie Diäthyläther und Tetrahydrofuran.

Die erfindungsgemäßen Umsetzungen gemäß Verfahrensvarianten (c) und (d) werden in Gegenwart eines Katalysators durchgeführt. Man kann alle üblicherweise verwendbaren Hydrierungskatalysatoren verwenden. Hierzu gehören vorzugsweise Edelmetall-, Edelmetalloxid- (bzw. Edelmetallhydroxid-) -Katalysatoren oder sogenannte 'Raney-Katalysatoren', wie insbesondere Platin, Platinoxid, Nickel, Rhodium, Rhodiumoxid, Ruthenium, Palladium und Osmium.

Die Reaktionstemperaturen können bei den Verfahrensvarianten (c) und (d) in einem größeren Bereich variiert werden. Im allgemeinen arbeitet man zwischen 20 und 200°C, vorzugsweise bei 80 bis 150°C.

Die Umsetzungen gemäß Verfahrensvarianten (c) und (d) können bei Normaldruck, aber auch bei erhöhtem Druck, vorzugsweise bei 1 bis 200 atü, durchgeführt werden.

Bei der Durchführung der erfindungsgemäßen Verfahrensvarianten (c) und (d) setzt man auf 1 Mol der Verbindungen der Formeln Va, Vb, Vc, bzw. VI vorzugsweise 2 Mol Wasserstoff und 0,01—0,1 Mol Katalysator ein. Zur Isolierung der Endprodukte wird vom Katalysator abfiltriert, gegebenenfalls vom Lösungsmittel im Vakuum befreit und die erhaltenen Produkte der Formel I werden durch Destillation oder Umkristallisation gereinigt.

Die erfindungsgemäßen Verbindungen der Formel I können gegebenenfalls in verschiedenen geometrischen Isomeren vorliegen, die in unterschiedlichen Mengenverhältnissen anfallen können. Außerdem liegen sie jeweils als optische Isomere vor. Dabei kann der Fall auftreten, daß bestimmte Isomere eine größere Wirksamkeit als andere aufweisen, so daß es gegebenenfalls zweckmäßig ist, die aktivere Komponete herzustellen bzw. zu isolieren, Sämtliche Isomeren werden erfindungsgemäß beansprucht.

Als Beispiele für besonders wirksame Vertreter der erfindungsgemäßen Wirkstoffe seien außer der Herstellungsbeispielen und den Beispielen der Tabelle 1 genannt:

2-Benzyloxymethyl-5-methyl-tetrahydrofuran
2-(2-Fluorbenzyloxymethyl)-5-methyl-tetrahydrofuran
2-(2-Chlorbenzyloxymethyl)-5-methyl-tetrahydrofuran
2-(2-Brombenzyloxymethyl)-5-methyl-tetrahydrofuran
2-(2-Methylbenzyloxymethyl)-5-methyl-tetrahydrofuran
2-(4-Fluorbenzyloxymethyl)-5-methyl-tetrahydrofuran
2-(2,6-Dichlorbenzyloxymethyl)-5-methyl-tetrahydrofuran
2-Benzyloxymethyl-2-methyl-tetrahydrofuran
2-(2-Fluorbenzyloxymethyl)-2-methyl-tetrahydrofuran
2-(2-Chlorbenzyloxymethyl)-2-methyl-tetrahydrofuran
2-(2-Brombenzyloxymethyl)-2-methyl-tetrahydrofuran
2-(2-Methylbenzyloxymethyl)-2-methyl-tetrahydrofuran
2-(4-Fluorbenzyloxymethyl)-2-methyl-tetrahydrofuran
2-(2,6-Dichlorbenzyloxymethyl)-2-methyl-tetrahydrofuran
2-Benzyloxymethyl-2-äthyl-tetrahydrofuran
2-(2-Fluorbenzyloxymethyl)-2-äthyl-tetrahydrofuran
2-(2-Chlorbenzyloxymethyl)-2-äthyl-tetrahydrofuran
2-(2-Brombenzyloxymethyl)-2-äthyl-tetrahydrofuran
2-(2-Methylbenzyloxymethyl)-2-äthyl-tetrahydrofuran
2-(4-Fluorbenzyloxymethyl)-2-äthyl-tetrahydrofuran
2-(2,6-Dichlorbenzyloxymethyl)-2-äthyl-tetrahydrofuran
2-Benzyloxymethyl-2-propyl-tetrahydrofuran

2-(2-Fluorbenzyloxymethyl)-2-propyl-tetrahydrofuran
2-(2-Chlorbenzyloxymethyl)-2-propyl-tetrahydrofuran
2-(2-Brombenzyloxymethyl)-2-propyl-tetrahydrofuran
2-(2-Methylbenzyloxymethyl)-2-propyl-tetrahydrofuran
2-(4-Fluorbenzyloxymethyl)-2-propyl-tetrahydrofuran
2-(2,6-Dichlorbenzyloxymethyl)-2-propyl-tetrahydrofuran
2-Benzyloxymethyl-2,5,5-trimethyl-tetrahydrofuran
2-(2-Fluorbenzyloxymethyl)-2,5,5-trimethyl-tetrahydrofuran
2-(2-Chlorbenzyloxymethyl)-2,5,5-trimethyl-tetrahydrofuran
2-(2-Brombenzyloxymethyl)-2,5,5-trimethyl-tetrahydrofuran
2-(2-Methylbenzyloxymethyl)-2,5,5-trimethyl-tetrahydrofuran
2-(2,6-Dichlorbenzyloxymethyl)-2,5,5-trimethyl-tetrahydrofuran
2-Benzyloxymethyl-2-äthyl-5,5-dimethyl-tetrahydrofuran
2-(2-Fluorbenzyloxymethyl)-2-äthyl-5,5-dimethyl-tetrahydrofuran
2-(2-Chlorbenzyloxymethyl)-2-äthyl-5,5-dimethyl-tetrahydrofuran
2-(2-Brombenzyloxymethyl)-2-äthyl-5,5-dimethyl-tetrahydrofuran
2-(2-Methylbenzyloxymethyl)-2-äthyl-5,5-dimethyl-tetrahydrofuran
2-(4-Fluorbenzyloxymethyl)-2-äthyl-5,5-dimethyl-tetrahydrofuran
2-(2,6-Dichlorbenzyloxymethyl)-2-äthyl-5,5-dimethyl-tetrahydrofuran
2-Benzyloxymethyl-2-propyl-5,5-dimethyl-tetrahydrofuran
2-(2-Fluorbenzyloxymethyl)-2-propyl-5,5-dimethyl-tetrahydrofuran
2-(2-Chlorbenzyloxymethyl)-2-propyl-5,5-dimethyl-tetrahydrofuran
2-(2-Fluorbenzyloxymethyl)-5-chlormethyl-tetrahydrofuran
2-(2-Chlorbenzyloxymethyl)-5-chlormethyl-tetrahydrofuran
2-(2-Brombenzyloxymethyl)-5-chlormethyl-tetrahydrofuran
2-(2-Brombenzyloxymethyl)-2-propyl-5,5-dimethyl-tetrahydrofuran
2-(2-Methylbenzyloxymethyl)-2-propyl-5,5-dimethyl-tetrahydrofuran
2-(4-Fluorbenzyloxymethyl)-2-propyl-5,5-dimethyl-tetrahydrofuran
2-(2,6-Dichlorbenzyloxymethyl)-2-propyl-5,5-dimethyl-tetrahydrofuran
2-Benzyloxymethyl-2-phenyl-tetrahydrofuran
2-(2-Fluorbenzyloxymethyl)-2-phenyl-tetrahydrofuran
2-(2-Chlorbenzyloxymethyl)-2-phenyl-tetrahydrofuran
2-(2-Brombenzyloxymethyl)-2-phenyl-tetrahydrofuran
2-(2-Methylbenzyloxymethyl)-2-phenyl-tetrahydrofuran
2-(4-Fluorbenzyloxymethyl)-2-phenyl-tetrahydrofuran
2-(2,6-Dichlorbenzyloxymethyl)-2-phenyl-tetrahydrofuran
2-Benzyloxymethyl-2-äthyl-5-phenyl-tetrahydrofuran
2-(2-Fluorbenzyloxymethyl)-2-äthyl-5-phenyl-tetrahydrofuran
2-(2-Chlorbenzyloxymethyl)-2-äthyl-5-phenyl-tetrahydrofuran
2-(2-Brombenzyloxymethyl)-2-äthyl-5-phenyl-tetrahydrofuran
2-(2-Methylbenzyloxymethyl)-2-äthyl-5-phenyl-tetrahydrofuran
2-(4-Fluorbenzyloxymethyl)-2-äthyl-5-phenyl-tetrahydrofuran
2-(2,6-Dichlorbenzyloxymethyl)-2-äthyl-5-phenyl-tetrahydrofuran
2-Benzyloxymethyl-2,5-diäthyl-5-methyl-tetrahydrofuran
2-(2-Fluorbenzyloxymethyl)-2,5-diäthyl-5-methyl-tetrahydrofuran
2-(2-Chlorbenzyloxymethyl)-2,5-diäthyl-5-methyl-tetrahydrofuran
2-(2-Brombenzyloxymethyl)-2,5-diäthyl-5-methyl-tetrahydrofuran
2-(2-Methylbenzyloxymethyl)-2,5-diäthyl-5-methyl-tetrahydrofuran
2-(4-Fluorbenzyloxymethyl)-2,5-diäthyl-5-methyl-tetrahydrofuran
2-(2,6-Dichlorbenzyloxymethyl)-2,5-diäthyl-5-methyl-tetrahydrofuran
2-(2-Methylbenzyloxymethyl)-5-chlormethyl-tetrahydrofuran
2-(4-Fluorbenzyloxymethyl)-5-chlormethyl-tetrahydrofuran
2-(2,6-Dichlorbenzyloxymethyl)-5-chlormethyl-tetrahydrofuran
2-Benzyloxymethyl-2-methyl-5-phenyl-tetrahydrofuran
2-(2-Fluorbenzyloxymethyl)-2-methyl-5-phenyl-tetrahydrofuran
2-(2-Chlorbenzyloxymethyl)-2-methyl-5-phenyl-tetrahydrofuran
2-(2-Brombenzyloxymethyl)-2-methyl-5-phenyl-tetrahydrofuran
2-(2-Methylbenzyloxymethyl)-2-methyl-5-phenyl-tetrahydrofuran
2-(4-Fluorbenzyloxymethyl)-2-methyl-5-phenyl-tetrahydrofuran
2-(2,6-Dichlorbenzyloxymethyl)-2-methyl-5-phenyl-tetrahydrofuran
2-Benzyloxymethyl-5-äthyl-tetrahydrofuran
2-(2-Fluorbenzyloxymethyl)-5-äthyl-tetrahydrofuran
2-(2-Chlorbenzyloxymethyl)-5-äthyl-tetrahydrofuran
2-(2-Brombenzyloxymethyl)-5-äthyl-tetrahydrofuran
2-(2-Methylbenzyloxymethyl)-5-äthyl-tetrahydrofuran

2-(4-Fluorbenzyloxymethyl)-5-äthyl-tetrahydrofuran
2-(2,6-Dichlorbenzyloxymethyl)-5-äthyl-tetrahydrofuran
2-(Benzyloxymethyl)-2,5-dimethyl-5-vinyl-tetrahydrofuran
2-(2-Fluorbenzyloxymethyl)-2,5-dimethyl-5-vinyl-tetrahydrofuran
2-(2-Chlorbenzyloxymethyl)-2,5-dimethyl-5-vinyl-tetrahydrofuran
2-(2-Brombenzyloxymethyl)-2,5-dimethyl-5-vinyl-tetrahydrofuran
2-(2-Methylbenzyloxymethyl)-2,5-dimethyl-5-vinyl-tetrahydrofuran
2-(4-Fluorbenzyloxymethyl)-2,5-dimethyl-5-vinyl-tetrahydrofuran
2-(2,6-Dichlorbenzyloxymethyl)-2,5-dimethyl-5-vinyl-tetrahydrofuran
2-(Benzyloxymethyl)-2,5-dimethyl-5-äthyl-tetrahydrofuran
2-(2-Fluorbenzyloxymethyl)-2,5-dimethyl-5-äthyl-tetrahydrofuran
2-(2-Chlorbenzyloxymethyl)-2,5-dimethyl-5-äthyl-tetrahydrofuran
2-(2-Brombenzyloxymethyl)-2,5-dimethyl-5-äthyl-tetrahydrofuran
2-(2-Methylbenzyloxymethyl)-2,5-dimethyl-5-äthyl-tetrahydrofuran
2-(4-Fluorbenzyloxymethyl)-2,5-dimethyl-5-äthyl-tetrahydrofuran
2-(2,6-Dichlorbenzyloxymethyl)-2,5-dimethyl-5-äthyl-tetrahydrofuran
2-(Benzyloxymethyl)-5-methoxymethyl-tetrahydrofuran
2-(2-Fluorbenzyloxymethyl)-5-methoxymethyl-tetrahydrofuran
2-(2-Chlorbenzyloxymethyl)-5-methoxymethyl-tetrahydrofuran
2-(2-Brombenzyloxymethyl)-5-methoxymethyl-tetrahydrofuran
2-(2-Methylbenzyloxymethyl)-5-methoxymethyl-tetrahydrofuran
2-(4-Fluorbenzyloxymethyl)-5-methoxymethyl-tetrahydrofuran
2-(2,6-Dichlorbenzyloxymethyl)-5-methoxymethyl-tetrahydrofuran
2-(Benzyloxymethyl)-5-chlormethyl-tetrahydrofuran

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
*Dikotyle Unkräuter* der Gattungen: Senf (Sinapsis), Kresse (Lepidium), Labkraut (Galium), Sternmiere (Stellaria), Kamille (Matricaria), Hundskamille (Anthemis), Knopfkraut (Galinsoga), Gänsefuß (Chenopodium), Brennessel (Urtica), Kreuzkraut (Senecio), Fuchsschwanz (Amaranthus), Portulak (Portulaca), Spitzklette (Xanthium), Winde (Convolvulus), Prunkwinde (Ipomoea), Knöterich (Polygonum), Sesbanie (Sesbania), Ambrosie (Ambrosia), Kratzdistel (Cirsium), Distel (Carduus), Gänsedistel (Sonchus), Nachtschatten (Solanum), Sumpfkresse (Rorippa), Rotala, Büchsenkraut (Lindernia), Taubnessel (Lamium), Ehrenpreis (Veronica), Schönmalve (Abutilon), Emex, Stechapfel (Datura), Veilchen (Viola), Hanfnessel, Hohlzahn (Galeopsis), Mohn (Papaver), Flockenblume (Centaurea).
*Dicotyle Kulturen* der Gattungen: Baumwolle (Gossypium), Sojabohne (Glycine), Rübe (Beta), Mohre (Daucus), Gartenbohne (Phaseolus), Erbse (Pisum), Kartoffel (Solanum), Lein (Linum), Prunkwinde (Ipomoea), Bohne (Vicia), Tabak (Nicotiana), Tomate (Lycopersicon), Erdnuß (Arachis), Kohl (Brassica), Lattich (Lactuca), Gurke (Cucumis), Kürbis (Cuburbita).
*Monokotyle Unkräuter* der Gattungen: Hühnerhirse (Echinochloa), Borstenhirse (Setaria), Hirse (Panicum), Fingerhirse (Digitaria), Lieschgras (Phleum), Rispengras (Poa), Schwingel (Festuca), Eleusine, Brachiaria, Lolch (Lolium), Trespe (Bromus), Hafer (Avena), Zypergras (Cyperus), Mohrenhirse (Sorghum), Quecke (Agropyron), Hundszahngras (Cynodon), Monocharia, Fimbristylis, Pfeilkraut (Sagittaria), Sumpfried (Eleocharis), Simse (Scirpus), Paspalum, Ischaemum, Sphenoclea, Dactyloctenium, Straußgras (Agrostis), Fuchsschwanzgras (Alopecurus), Windhalm (Apera).
*Monokotyle Kulturen* der Gattungen: Reis (Orzya), Mais (Zea), Weizen (Triticum), Gerste (Hordeum), Hafer (Avena), Roggen (Secale), Mohrenhirse (Sorghum), Hirse (Panicum), Zuckerrohr (Saccharum), Ananas (Ananas), Spargel (Asparagus), Lauch (Allium).

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe weisen insbesondere starke herbizide Wirkungen gegen Gräser auf, ohne verschiedene Kulturpflanzen zu schädigen. Die können deshalb vorzugsweise zur selektiven Ungräserbekämpfung eingesetzt werden. Als Kulturen kommen insbesondere infrage: Rüben, Sojabohnen, Bohnen, Baumwolle, Raps, Erdnüsse, Gemüse, Mais und Reis.

Die erfindungsgemäßen Wirkstoffe können in die üblichen Formulierungen übergeführt werden,

wie Lösungen, Emulsionen, Suspensionen, Pulver, Pasten und Granulate. Diese werden in bekannter Weise hergestellt, z.B. durch Vermischen der Wirkstoffe mit Streckmitteln, also flüssigen Lösungsmitteln, unter Druck stehenden verflüssigen Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, Benzol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Dichlordifluormethan oder Trichlorfluormethan; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als Emulgiermittel; nichtionogene und anionische Emulgatoren, wie Polyoxyäthylen-Fettsäure-Ester, Polyoxyäthylen-Fettalkohol-Äther, z.B. Alkylaryl-polyglycol-Äther, Alkylsulfonate, Alkylsulfate, Arylsulfonate sowie Eiweißhydrolysate; als Dispergiermittel: z.B. Lignin — Sulfitablaugen und Methylcellulose.

Die erfindungsgemäßen Wirkstoffe können als solche oder in ihren Formulierungen zur Verstärkung und Ergänzung ihres Wirkungsspektrums je nach beabsichtigter Verwendung mit anderen herbiziden Wirkstoffen kombiniert werden, wobei Fertigformulierung oder Tankmischung möglich ist.

Die Formulierungen enthalten im allgemeinen zwischen 0,1 und 95 Gewichtsprozent Wirkstoff, vorzugsweise zwischen 0,5 und 90 Gewichtsprozent.

Die Wirkstoffe können als solche, in Form ihrer Formulierungen oder der daraus bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Spritzen, Sprühen, Stäuben, Streuen und Gießen.

Die erfindungsgemäßen Wirkstoffe können sowohl nach als auch insbesondere vor dem Auflaufen der Pflanzen appliziert werden. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,2 und 6 kg/ha.

Die erfindungsgemäßen Wirkstoffe besitzen nicht nur herbizide Eigenschaften, sondern darüberhinaus auch eine fungizide und insektizide Wirksamkeit.

Die guten herbiziden Wirkungen der erfindungsgemäßen Wirkstoffe und ihre selektiven Einsatzmöglichkeiten gehen aus den nachfolgenden Beispielen hervor.

## Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate gehen aus der nachfolgenden Tabelle hervor:

TABELLE A

Pre-emergence Test

| Wirkstoffe | Wirkstoff-aufwand kg/ha | Zuckerrüben | Sojabohnen | Mais | Raps | Echinochloa crus galli | Alopecurus myosuroides | Poa annua |
|---|---|---|---|---|---|---|---|---|
| 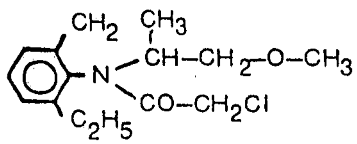 (bekannt) | 1,5 | 10 | 30 | 20 | 30 | 90 | 50 | 70 |
| | 1,0 | 10 | 20 | 10 | 10 | 90 | 40 | 50 |
| 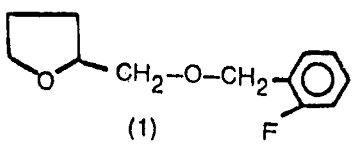 (1) | 1,5 | 0 | 0 | 0 | 0 | 100 | 60 | 90 |
| | 1,0 | 0 | 0 | 0 | 0 | 90 | 40 | 80 |

*Herstellungsbeispiele*

Beispiel 1

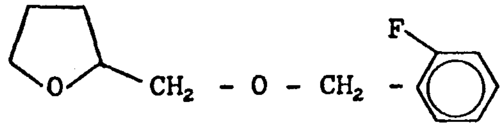 (1)

(Verfahrenzvariante a)

Zu einem Gemisch von 4,8 g (0,2 Mol) Natriumhydrid (6,0 g 80%iges Natriumhydrid in Paraffinöl) in 200 ml absolutem Dioxan werden bei Raumtemperatur unter Rühren 20,4 g (0,2 Mol) Tetrahydrofurfurylalkohol zugetropft. Man erhitzt danach noch 30 Minuten unter Rückfluß, kühlt auf 50°C ab und tropft dann zu dem so erhaltenen Natriumsalz 38 g (0,2 Mol) 2-Fluorbenzylbromid zu. Anschließend erhitzt man noch 3 Stunden unter Rückfluß, läßt auf Raumtemperatur abkühlen, versetzt zur Zerstörung von überschüssigem Natriumhydrid mit 20 ml Methanol und engt durch Abdestillieren des Lösungsmittels im Vakuum ein. Der Rückstand wird in 200 ml Wasser aufgenommen und mit Methylenchlorid extrahiert. Die organische Phase wird über Natriumsulfat getrocknet, filtriert, das Lösungsmittel abgezogen und der Rückstand im Vakuum fraktioniert. Man erhält 37,8 g (90% der Theorie) 2-(2-Fluorbenzyloxymethyl)-tetrahydrofuran vom Siedepunkt 79°C/0,1 mm.

Beispiel 2

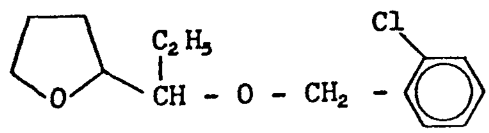 (2)

(Verfahrensvariante d)

25 g (0,1 Mol) 2-[1-(2-Chlorbenzyloxy)-propyl]-furan werden in 200 ml Methanol gelöst und nach Zugabe von 5 g Rhodium-Katalysator (5% Rhodium auf Aluminiumoxid) 4 Stunden bei 5 atü und Raumtemperatur hydriert. Danach wird der Katalysator abfiltriert, das Filtrat durch Abdestillieren des Lösungsmittels im Vakuum eingeengt und der Rückstand fraktioniert im Vakuum destilliert. Man erhält 15,7 g (62% der Theorie) 2-[1-(2-Chlorbenzyloxy)-propyl]-tetrahydrofuran vom Siedepunkt 101—105°C/0,1 mm.

In analoger Weise können die in der nachfolgenden Tabelle 1 aufgeführten Verbindungen hergestellt werden.

TABELLE 1

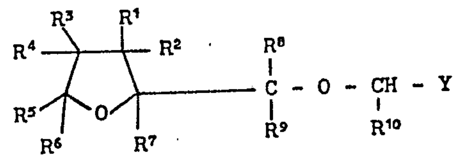

| Bsp. Nr. | $R^1$ | $R^2$ | $R^3$ | $R^4$ | $R^5$ | $R^6$ | $R^7$ | $R^8$ | $R^9$ | $R^{10}$ | Y | Schmelzpunkt (°C) bzw. Siedepunkt (°C) /mmHg-Säule |
|---|---|---|---|---|---|---|---|---|---|---|---|---|
| 3 | H | H | H | H | H | H | H | H | H | H | 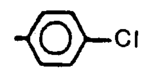 | 150/0,1 |
| 4 | H | H | H | H | H | H | H | H | H | H | 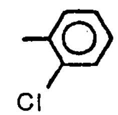 | 110/0,1 |
| 5 | H | H | H | H | H | H | H | H | H | H | 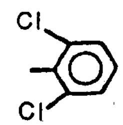 | 120/0,1 |
| 6 | H | H | H | H | H | H | H | H | H | H | 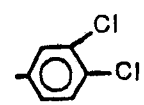 | 112/0,1 |
| 7 | H | H | H | H | H | H | H | H | H | H | 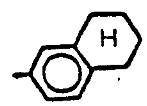 | 134/0,1 |
| 8 | H | H | H | H | H | H | H | H | H | H | 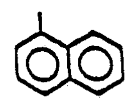 | 155/0,4 |

TABELLE 1 (Fortsetzung)

| Bsp. Nr. | $R^1$ | $R^2$ | $R^3$ | $R^4$ | $R^5$ | $R^6$ | $R^7$ | $R^8$ | $R^9$ | $R^{10}$ | Y | Schmelzpunkt (°C) bzw. Siedepunkt (°C) /mmHg-Säule |
|---|---|---|---|---|---|---|---|---|---|---|---|---|
| 9 | H | H | H | H | H | H | H | H | H | H | 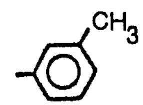 | 96 /0,4 |
| 10 | H | H | H | H | H | H | H | H | H | H | 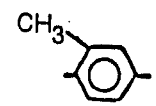 | 94 /0,3 |
| 11 | H | H | H | H | H | H | H | H | H | H | 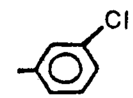 | 103 /0,3 |
| 12 | H | H | H | H | H | H | H | H | H | H | 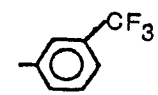 | 126 /0,1 |
| 13 | H | H | H | H | H | H | H | H | H | H | 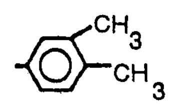 | 100 /0,2 |
| 14 | H | H | H | H | H | H | H | H | H | H | 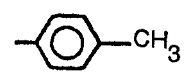 | 116 /0,2 |
| 15 | H | H | H | H | H | H | H | H | H | H | 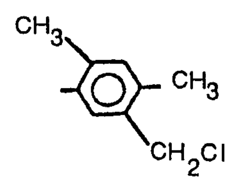 | 156 /0,3 |

TABELLE 1 (Fortsetzung)

| Bsp. Nr. | $R^1$ | $R^2$ | $R^3$ | $R^4$ | $R^5$ | $R^6$ | $R^7$ | $R^8$ | $R^9$ | $R^{10}$ | Y | Schmelzpunkt (°C) bzw. Siedepunkt (°C) /mmHg-Säule |
|---|---|---|---|---|---|---|---|---|---|---|---|---|
| 16 | H | H | H | H | H | H | H | H | H | H | 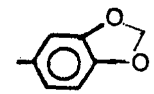 | 131 /0,1 |
| 17 | H | H | H | H | H | H | H | H | H | H | 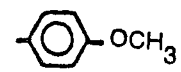 | 125 /0,1 |
| 18 | H | H | H | H | H | H | H | H | H | H | 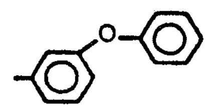 | 158 /0,1 |
| 19 | H | H | H | H | H | H | H | H | H | H | 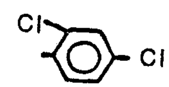 | 125 /0,1 |
| 20 | H | H | H | H | H | H | H | H | H | H | 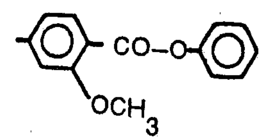 | 105 /0,1 |
| 21 | H | H | H | H | H | H | H | H | H | H | 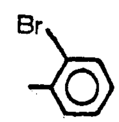 | 107 /0,1 |
| 22 | H | H | H | H | H | H | H | H | H | H | 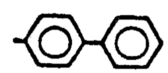 | 160 /0,1 |

TABELLE 1 (Fortsetzung)

| Bsp. Nr. | $R^1$ | $R^2$ | $R^3$ | $R^4$ | $R^5$ | $R^6$ | $R^7$ | $R^8$ | $R^9$ | $R^{10}$ | Y | Schmelzpunkt (°C) bzw. Siedepunkt (°C) / mmHg-Säule |
|---|---|---|---|---|---|---|---|---|---|---|---|---|
| 23 | H | H | H | H | H | H | H | H | $C_2H_5$ | H | 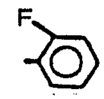 | 88 /0,1 |
| 24 | H | H | H | H | H | H | H | H | $CH_3$ | H | 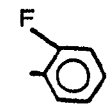 | 90 /0,6 |
| 25 | H | H | H | H | H | H | H | H | $CH_3$ | H | 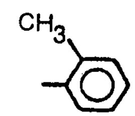 | 93 /0,4 |
| 26 | H | H | H | H | H | H | H | $CH_3$ | H | H | 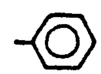 | 101 /0,5 |
| 27 | H | H | H | H | H | H | H | $C_2H_5$ | $C_2H_5$ | H | 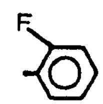 | 110 /0,4 |
| 28 | H | H | H | H | H | H | H | $C_2H_5$ | $C_2H_5$ | H | 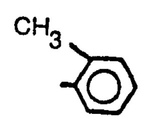 | 99–103 /0,4 |
| 29 | H | H | H | H | H | H | H | $C_2H_5$ | $C_2H_5$ | H | 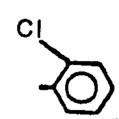 | 108 /0,1 |

TABELLE 1 (Fortsetzung)

| Bsp. Nr. | $R^1$ | $R^2$ | $R^3$ | $R^4$ | $R^5$ | $R^6$ | $R^7$ | $R^8$ | $R^9$ | $R^{10}$ | Y | Schmelzpunkt (°C) bzw. Siedepunkt (°C)/mmHg-Säule |
|---|---|---|---|---|---|---|---|---|---|---|---|---|
| 30 | H | H | H | H | H | H | H | H | $CH_3$ | H | 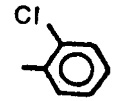 | 105/0,1 |
| 31 | H | H | H | H | H | H | H | H | 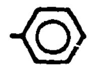 | H | 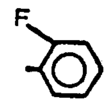 | 143/0,1 |
| 32 | H | H | H | H | H | H | H | 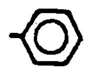 | H | H | 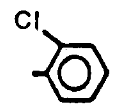 | 159/0,1 |
| 33 | H | H | H | H | H | H | H | $CH_3$ | $CH_3$ | H | 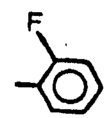 | 80–83/0,1 |
| 34 | H | H | H | H | H | H | H | H | H | 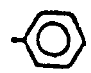 | 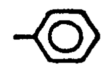 | 143/0,06 |
| 35 | H | H | H | H | H | H | H | 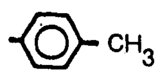 | H | H | 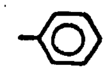 | 148/0,3 |
| 36 | H | H | H | H | H | H | H | 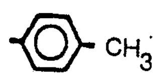 | H | H | 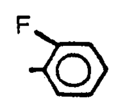 | 151/,03 |

TABELLE 1 (Fortsetzung)

| Bsp. Nr. | $R^1$ | $R^2$ | $R^3$ | $R^4$ | $R^5$ | $R^6$ | $R^7$ | $R^8$ | $R^9$ | $R^{10}$ | Y | Schmelzpunkt (°C) bzw. Siedepunkt (°C) /mmHg-Säule |
|---|---|---|---|---|---|---|---|---|---|---|---|---|
| 37 | H | H | H | H | H | H | H | 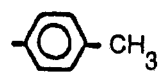 | H | H | 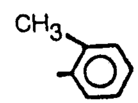 | 148 /0,5 |
| 38 | H | H | H | H | $CH_3$ | $CH_3$ | H | H | H | H | 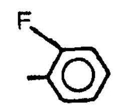 | Oel |

*Herstellung von Ausgangsprodukten*

(a) Das gemäß Beispiel 2 als Ausgangsprodukt eingesetzte 2-[1-(2-Chlorbenzyloxy)-propyl]-furan kann wie folgt hergestellt werden:

Zu einem Gemisch von 4,8 g (0,2 Mol) Natriumhydrid (6,0 g 80%iges Natriumhydrid in Paraffinöl) und 200 ml absolutem Dioxan werden bei Raumtemperatur 27,2 g (0,2 Mol) 2-(1-Hydroxypropyl)-furan zugetropft.

Man erhitzt danach 30 Min. unter Rückfluß, kühlt auf 50°C ab und tropft dann zu dem so erhaltenen Natriumsalz 32 g (0,2 Mol) 2-Chlorbenzylchlorid zu. Anschließend erhitzt man noch 3 Stunden unter Rückfluß, versetzt zur Zerstörung von überschüssigem Natriumhydrid mit 20 ml Methanol und engt durch Abdestillieren des Lösungsmittels im Vakuum ein.

Der Rückstand wird in 200 ml Wasser aufgenommen und mit Methylenchlorid extrahiert. Die organische Phase wird über Natriumsulfat getrocknet, filtriert, das Lösungsmittel abgezogen und der Rückstand im Vakuum fraktioniert. Man erhält 36,0 g (72% d. Th.) 2-[1-(2-Chlorbenzyloxy)-propyl]-furan vom Siedepunkt 95—97°C/0,1 mm. Hg.

**Patentansprüche**

1. Tetrahydrofuran-Derivate der Formel I

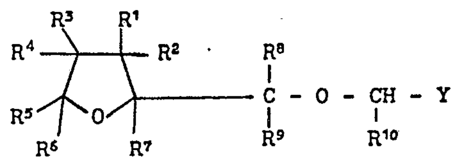

(I)

in welcher

$R^1$ bis $R^6$ gleich oder verschieden sind und für Wasserstoff, geradkettiges oder verzweigtes $C_{1-6}$-Alkyl, $C_{1-2}$-Halogenalkyl mit bis zu drei gleichen oder verschiedenen Halogenatomen, Alkoxyalkyl mit 1 oder 2 C-Atomen in jedem Alkylteil, sowie für jeweils gegebenenfalls durch Halogen, $C_{1-2}$-Alkyl, $C_{1-2}$-Alkoxy oder $C_{1-2}$-Halogenalkyl mit bis zu drei gleichen oder verschiedenen Halogenatomen substituiertes Phenyl oder Benzyloxyalkyl mit 1 oder 2 C-Atomen im Alkylteil stehen,

$R^5$ und $R^6$ auch gemeinsam für einen gesättigten carbocyclischen Ring mit insgesamt 3 bis 6 C-Atomen stehen,

$R^7$ für Wasserstoff, geradkettiges oder verzweigtes $C_{1-6}$-Alkyl, $C_{3-6}$-Cycloalkyl, $C_{2-4}$-Alkenyl, $C_{2-4}$-Alkinyl, Alkoxyalkyl mit 1 bis 2 C-Atomen in jedem Alkylteil, sowie für jeweils durch Halogen, $C_{1-2}$-Alkyl, $C_{1-2}$-Alkoxy, oder $C_{1-2}$-Halogenalkyl mit bis zu drei gleichen oder verschiedenen Halogenatomen substituiertes Phenyl oder Benzyloxyalkyl mit 1 oder 2 C-Atomen im Alkylteil steht,

$R^8$ und $R^9$ gleich oder verschieden sind und für Wasserstoff, geradkettiges oder verzweigtes $C_{1-6}$-Alkyl, $C_{2-4}$-Alkenyl, $C_{2-4}$-Alkinyl, $C_{3-6}$-Cycloalkyl, $C_{1-4}$-Alkoxy, sowie für jeweils gegebenenfalls durch Halogen, $C_{1-2}$-Alkyl, $C_{1-2}$-Alkoxy oder $C_{1-2}$-Halogenalkyl mit bis zu drei gleichen oder verschiedenen Halogenatomen substituiertes Phenyl, Benzyl oder Benzyloxy stehen,

$R^{10}$ für Wasserstoff, geradkettiges oder verzweigtes $C_{1-6}$-Alkyl, $C_{2-4}$-Alkenyl, $C_{2-4}$-Alkinyl, $C_{1-2}$-Halogenalkyl mit bis zu drei gleichen oder verschiedenen Halogenatomen, sowie für gegebenenfalls durch Halogen, $C_{1-2}$-Alkyl, $C_{1-2}$-Alkoxy oder $C_{1-2}$-Halogenalkyl mit bis zu drei gleichen oder verschiedenen Halogenatomen substituiertes Phenyl steht und

Y für gegebenenfalls substituiertes Aryl mit 6 bis 10 C-Atomen steht, welches einen oder mehrere gleiche oder verschiedene der folgenden Substituenten tragen kann: die Halogene Fluor, Chlor oder Brom; Alkyl und Alkoxy mit jeweils 1 bis 4 C-Atomen; Halogenalkyl, Halogenalkoxy und Halogenalkylthio mit jeweils bis zu 4 C-Atomen und bis zu 5 Halogenatomen; gegebenenfalls durch Halogen, Alkyl und Alkoxy mit jeweils 1 bis 2 C-Atomen sowie Halogenalkyl mit bis zu 2 C-Atomen und bis zu 3 gleichen oder verschiedenen Halogenatomen, substituiertes Phenyl, Phenoxy oder Phenoxycarbonyl; Alkoxycarbonyl mit 1 bis 4 C-Atomen im Alkylteil; die Methylendioxy-Gruppe sowie den Tri-, Tetra- oder Pentamethylen-Rest; mit der Maßgabe, daß, falls Y für Phenyl steht, dieses substituiert sein muß, wenn $R^1$ bis $R^{10}$ Wasserstoff bedeuten.

2. Verfahren zur Herstellung von Tetrahydrofuran-Derivaten der Formel I; dadurch gekennzeichnet, daß man

(a) Alkoholate von 2-Hydroxymethyl-tetrahydrofuran-Derivaten der Formel II

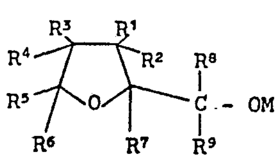

(II)

in welcher

$R^1$ bis $R^9$ die oben angegebene Bedeutung haben und
M für ein Alkali- oder Erdalkalimetall steht,
mit einer Verbindung der Formel III

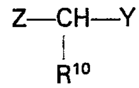

(III)

in welcher
$R^{10}$ und Y die oben angegebene Bedeutung haben und
Z für Halogen den Mesylat- oder Tosylat-Rest steht,
in an sich bekannter Weise, gegebenenfalls in Gegenwart eines Verdünnungsmittels umsetzt, oder daß man

(b) Diole der Formel IV

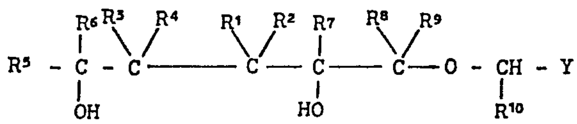

(IV)

in welcher
$R^1$ bis $R^{10}$ und
Y die oben angegebene Bedeutung haben,
in an sich bekannter Weise in Gegenwart eines sauren Katalysators und gegebenenfalls in Gegenwart eines Verdünnungsmittels erhitzt; oder daß man

(c) diejenigen Verbindungen der Formel I, in denen entweder $R^1$ und $R^7$ oder $R^1$ und $R^4$ oder $R^4$ und $R^5$ für Wasserstoff stehen, dadurch erhält, daß man Dihydrofuran-Derivate der Formeln Va, Vb und Vc

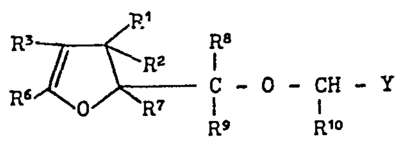

(Va)

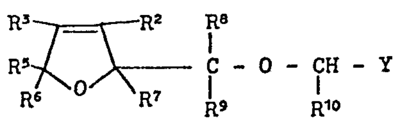

(Vb)

und/oder

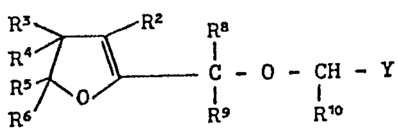

(Vc)

in welchen
$R^1$ bis $R^{10}$ und
Y die oben angegebene Bedeutung haben,
in an sich bekannter Weise in Gegenwart eines Katalysators und gegebenenfalls in Gegenwart eines Verdünnungsmittels mit Wasserstoff hydriert; oder daß man

(d) diejenigen Verbindungen der Formel I, in denen $R^1$, $R^4$, $R^5$ und $R^7$ für Wasserstoff stehen, dadurch erhält, daß man Furan-Derivate der Formel VI

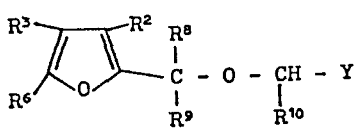

(VI)

in welcher
$R^2$, $R^3$, $R^6$, $R^8$, $R^9$, $R^{10}$ und Y die oben angegebene Bedeutung haben,
in an sich bekannter Weise in Gegenwart eines Katalysators und gegebenenfalls in Gegenwart eines Verdünnungsmittels mit Wasserstoff hydriert.

3. Herbizide Mittel, gekennzeichnet durch einen Gehalt an Tetrahydrofuran-Derivaten gemäß Anspruch 1.

4. Verwendung von Tetrahydrofuran-Derivaten gemäß Anspruch 1 zur Bekämpfung von unerwünschtem Pflanzenwachstum.

5. Verfahren zur Herstellung von herbiziden Mitteln, dadurch gekennzeichnet, daß man Tetrahydrofuran-Derivate gemäß Anspruch 1 mit Streckmitteln und/oder oberflächenaktiven Mitteln vermischt.

**Revendications**

1. Dérivés du tétrahydrofuranne de formule I

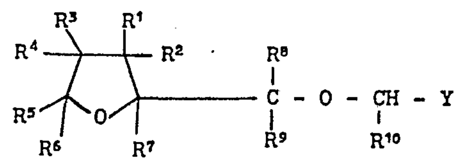

(I)

dans laquelle

$R^1$ à $R^6$, identiques ou différents, représentent l'hydrogène, un groupe alkyle en $C_1$—$C_6$ à chaîne droite ou ramifiée, halogénoalkyle en $C_1$—$C_2$ contenant jusqu'à 3 atomes d'halogènes identiques ou différents, alcoxyalkyle contenant 1 ou 2 atomes de carbone dans chacune des parties alkyle, ou encore phényle ou benzyloxyalkyle contenant 1 ou 2 atomes de carbone dans la partie alkyle, ces deux derniers groupes pouvant le cas échéant être substitués par des halogènes, des groupes alkyle en $C_1$—$C_2$, alcoxy en $C_1$—$C_2$ ou halogénoalkyle en $C_1$—$C_2$ contenant jusqu'à 3 atomes d'halogènes identiques ou différents,

$R^5$ et $R^6$ forment également ensemble un noyau carbocyclique saturé contenant ou total 3 à 6 atomes de carbone,

$R^7$ représente l'hydrogène, un groupe alkyle en $C_1$—$C_6$ à chaîne droite ou ramifiée, cycloalkyle en $C_3$—$C_6$, alcényle en $C_2$—$C_4$, alcynyle en $C_2$—$C_4$, alcoxyalkyle contenant 1 ou 2 atomes de carbone dans chacune des parties alkyle, ou encore phényle ou benzyloxyalkyle contenant 1 ou 2 atomes de carbone dans la partie alkyle, ces deux derniers groupes étant substitués par des halogènes, des groupes alkyle en $C_1$—$C_2$, alcoxy en $C_1$—$C_2$ ou halogénoalkyle en $C_1$—$C_2$ contenant jusqu'à 3 atomes d'halogènes identiques ou différents,

$R^8$ et $R^9$ identiques ou différents, représentent l'hydrogène, un groupe alkyle en $C_1$—$C_6$ à chaîne droite ou ramifiée, alcényle en $C_2$—$C_4$, alcynyle en $C_2$—$C_4$, cycloalkyle en $C_3$—$C_6$, alcoxy en en $C_1$—$C_4$ ou bien phényle, benzyle ou benzyloxy, ces trois derniers groupes pouvant le cas échéant être substitués par des halogènes, des groupes alkyle en $C_1$—$C_2$, alcoxy en $C_1$—$C_2$ ou halogéno-alkyle en $C_1$—$C_2$ contenant jusqu'à 3 atomes d'halogènes identiques ou différents,

$R^{10}$ représente l'hydrogène, un groupe alkyle en $C_1$—$C_6$ à chaîne droite ou ramifiée, alcényle en $C_2$—$C_4$, alcynyle en $C_2$—$C_4$, halogénoalkyle en $C_1$—$C_2$ contenant jusqu'à 3 atomes d'halogène identiques ou différents, ou encore phényle éventuellement substitué par des halogènes, des groupes alkyle en $C_1$—$C_2$, alcoxy en $C_1$—$C_2$ ou halogénoalkyle en $C_1$—$C_2$ contenant jusqu'à 3 atomes d'halogènes identiques ou différents, et

Y représente un groupe aryle éventuellement substitué en $C_1$—$C_{10}$, qui peut porter un ou plusieurs substituants identiques ou différents parmi les suivants: les halogènes, fluor, chlore ou brome; les groupes alkyle et alcoxy contenant chacun 1 à 4 atomes de carbone; les groupes halogénoalkyle, halogénoalcoxy et halogénoalkylthio contenant chacun jusqu'à 4 atomes de carbone et jusqu'à 5 atomes d'halogènes; un groupe phényle éventuellement substitué par des halogènes, des groupes alkyle et alcoxy contenant chacun 1 ou 2 atomes de carbone ou halogénoalkyle contenant jusqu'à 2 atomes de carbone et jusqu'à 3 atomes d'halogènes identiques ou différents, phénoxy ou phénoxycarbonyle; un groupe alcoxycarbonyle contenant 1 à 4 atomes de carbone dans la partie alkyle; le groupe méthylène-dioxy ou le reste tri-, tétra- ou penta-méthylène; étant spécifié que, lorsque Y représente un groupe phényle, celui-ci doit être substitué si $R^1$ à $R^{10}$ représentent l'hydrogène.

2. Procédé de préparation des dérivés du tétrahydrofuranne de formule I, caractérisé en ce que: (a) on fait réagir des alcoolates de dérivés du 2-hydroxyméthyl-tétrahydrofuranne de formule

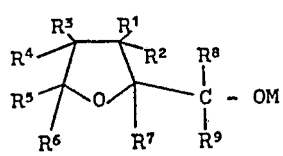

(II)

dans laquelle

$R^1$ à $R^9$ ont les significations indiquées ci-dessus, et

M représente un métal alcalin ou alcalino-terreux,

avec un composé de formule

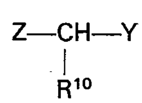

(III)

dans laquelle

$R^{10}$ et Y ont les significations indiquées ci-dessus, et

Z représente un halogène, le reste mésylate ou tosylate,

de manière connue en soi, éventuellement en présence d'un diluant, ou bien

(b) on chauffe des diols de formule

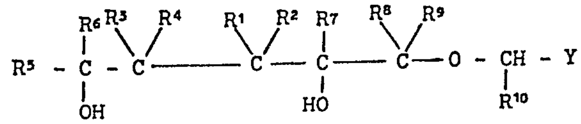 (IV)

dans laquelle

$R^1$ à $R^{10}$ et Y ont les significations indiquées ci-dessus,

de manière connue en soi, en présence d'un catalyseur acide et éventuellement en présence d'un diluant;

ou bien

(c) on obtient les composés de formule I dans laquelle ou bien $R^1$ et $R^7$, ou bien $R^1$ et $R^4$ ou bien $R^4$ et $R^5$ représentent l'hydrogène, en hydrogénant des dérivés du dihydrofuranne répondant aux formules

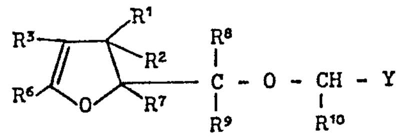 (Va)

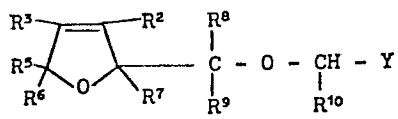 (Vb)

et/ou

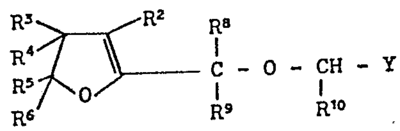 (Vc)

dans lesquelles

$R^1$ à $R^{10}$ et Y ont les significations indiquées ci-dessus,

de manière connue en soi, en présence d'un catalyseur et le cas échéant en présence d'un diluant, ou bien

(d) on obtient les composés de formule I dans laquelle $R^1$, $R^4$, $R^5$ et $R^7$ représentent l'hydrogène, en hydrogénant des dérivés du furanne de formule

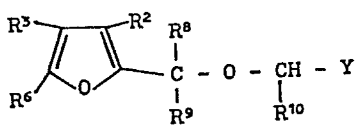 (VI)

dans laquelle

$R^2$, $R^3$, $R^6$, $R^8$, $R^9$, $R^{10}$ et Y ont les significations indiquées ci-dessus,

de manière connue en soi, en présence d'un catalyseur et le cas échéant en présence d'un diluant.

3. Produit herbicide caractérisé en ce qu'il contient des dérivés du tétrahydrofuranne selon la revendication 1.

4. Utilisation des dérivés du tétrahydrofuranne selon la revendication 1, pour combattre la croissance des végétaux indésirables.

5. Procédé de préparation de produits herbicides caractérisé en ce que l'on mélange des dérivés du tétrahydrofuranne selon la revendication 1, avec des diluants et/ou des produits tensioactifs.

**Claims**

1. Tetrahydrofurane derivatives of the formula I

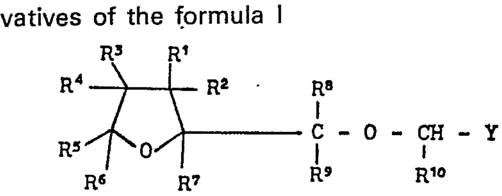 (I)

in which

$R^1$ to $R^6$ are identical or different and represent hydrogen, straight-chain or branched $C_{1-6}$ alkyl, $C_{1-2}$

halogenalkyl with up to three identical or different halogen atoms, alkoxyalkyl with 1 or 2 C atoms in each alkyl part, as well as phenyl or benzyloxyalkyl with 1 or 2 C atoms in the alkyl part each optionally substituted by halogen, $C_{1-2}$ alkyl, $C_{1-2}$ alkoxy or $C_{1-2}$ halogenoalkyl with up to three identical or different halogen atoms,

$R^5$ and $R^6$ together also represent a saturated carboxylic ring with altogether 3 to 6 C atoms,

$R^7$ represents hydrogen, straight-chain or branched $C_{1-6}$ alkyl, $C_{3-6}$ cycloalkyl, $C_{2-4}$ alkenyl, $C_{2-4}$ alkinyl, alkoxyalkyl with 1 to 2 C atoms in each alkyl part, as well as phenyl or benzyloxyalkyl with 1 or 2 C atoms in the alkyl part each substituted by halogen, $C_{1-2}$ alkyl, $C_{1-2}$ alkoxy, or $C_{1-2}$ halogenoalkyl with up to three identical or different halogen atoms,

$R^8$ and $R^9$ are identical or different and represent hydrogen, straight-chain or branched $C_{1-6}$ alkyl, $C_{2-4}$ alkenyl, $C_{2-4}$ alkinyl, $C_{3-6}$ cycloalkyl, $C_{1-4}$ alkoxy, as well as phenyl, benzyl or benzyloxy each optionally substituted by halogen, $C_{1-2}$ alkyl, $C_{1-2}$ alkoxy or $C_{1-2}$ halogenoalkyl with up to three identical or different halogen atoms,

$R^{10}$ represents hydrogen, straight-chain or branched $C_{1-6}$ alkyl, $C_{2-4}$ alkenyl, $C_{2-4}$ alkinyl, $C_{1-2}$ halogenoalkyl with up to three identical or different halogen atoms, as well as phenyl optionally substituted by halogen, $C_{1-2}$ alkyl, $C_{1-2}$ alkoxy or $C_{1-2}$ halogenoalkyl with up to three identical or different halogen atoms and

Y represents optionally substituted aryl with 6 to 10 C atoms, which can carry one or more identical or different substituents from the following: the halogens fluorine, chlorine or bromine; alkyl and alkoxy with in each case 1 to 4 C atoms; halogenoalkyl, halogenoalkoxy and halogenoalkylthio with in each case up to 4 C atoms and up to 5 halogen atoms; phenyl, phenoxy or phenoxycarbonyl optionally substituted by halogen, alkyl and alkoxy with in each case 1 to 2 C atoms as well as halogenalkyl with up to 1 C atoms and up to 3 identical or different halogen atoms as well as halogenoalkyl with up to 1 C atoms and up to 3 identical or different halogen the tri-, tetra- or penta-methylene radical; with the proviso that if Y represents phenyl this must be substituted if $R^1$ to $R^{10}$ denote hydrogen.

2. Process for the preparation of tetrahydrofurane derivatives of the formula (I), characterised in that

a) alcoholates of 2-hydroxymethyl-tetrahydrofurane derivatives of the formula

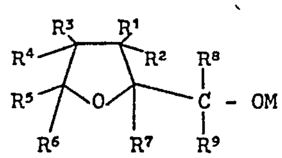

(II)

in which

$R^1$ to $R^9$ have the meaning indicated above and

M represents an alkali metal or alkaline earth metal,

are reacted with a compound of the formula

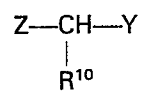

(III)

in which

$R^{10}$ and Y have the meaning indicated above and

Z represents halogen, or the mesylate or tosylate radical,

in a manner known per se optionally in the presence of a diluent, or in that

b) diols of the formula

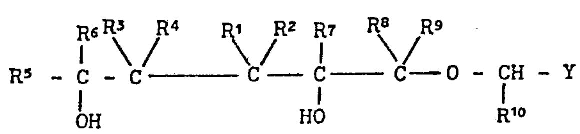

(IV)

in which

$R^1$ to $R^{10}$ and Y have the meaning indicated above,

are heated in a manner known per se in the presence of an acid catalyst and optionally in the presence of a diluent; or in that

(c) those compounds of the formula (I) in which either $R^1$ and $R^7$ or $R^1$ and $R^4$ or $R^4$ and $R^5$ represent hydrogen are obtained when dihydrofurane derivatives of the formulae

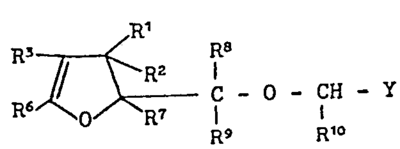

(Va)

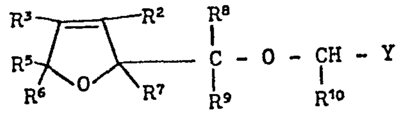 (Vb)

and/or

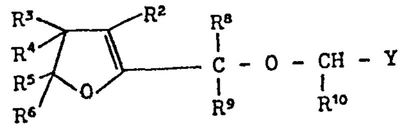 . (Vc)

in which
$R^1$ to $R^{10}$ and Y have the meaning indicated above,
are hydrogenated with hydrogen, in a manner known per se in the presence of a catalyst and optionally in the presence of a diluent; or in that

d) those compounds of the formula (I) in which $R^1$, $R^4$, $R^5$ and $R^7$ represent hydrogen, are obtained when furane derivatives of the formula

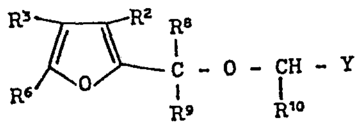 (VI)

in which
$R^2$, $R^3$, $R^6$, $R^8$, $R^9$, $R^{10}$ and Y have the meaning indicated above,
are hydrogenated with hydrogen, in a manner known per se, in the presence of a catalyst and optionally in the presence of a diluent.

3. Herbicidal agents, characterised in that they contain tetrahydrofurane derivatives according to Claim 1.

4. Use of tetrahydrofurane derivatives according to Claim 1 for combating undesired plant growth.

5. Process for the preparation of herbicidal agents, characterised in that tetrahydrofurane derivatives according to Claim 1 are mixed with extenders and/or surface-active agents.